# EUROPEAN PATENT APPLICATION

(11) **EP 4 186 490 A1**
(43) Date of publication of application: **31.05.2023**
(21) Application number: 21211003.5
(22) Date of filing: 29.11.2021
(51) Int. Cl.: A61K 9/00, A61K 9/02, A61K 9/107, A61K 31/00

(54) **KIT FOR PREPARING A MEDICAMENT, CANNABINOID COMPOSITION, AND PREPARATION METHOD**

(71) Applicant: Sanity Group GmbH, 10117 Berlin (DE)
(72) Inventor: Ternelli, Marco, 42021 Bibbiano (IT)
(74) Representative: Strehl Schübel-Hopf & Partner

(57) **Abstract**

A kit for preparing a medicament, wherein the kit does not contain the active ingredient of the medicament and contains components, equipment and parts for carrying out, after adding the active ingredient, a two-syringe emulsification to prepare an emulsion containing the active ingredient; and for preparing the medicament using the emulsion; a method of preparing a medicament containing a cannabinoid in an emulsion; and a medicament containing a cannabinoid in an emulsion.

## Description

### Field of the invention

The present invention relates to a kit for preparing a medicament, a method of preparing a medicament by using the two-syringe technique, a medicament containing cannabinoids in an emulsion, and a cannabinoid-containing medicament in the form of a nasal spray, a rectal suppository, or a vaginal suppository for use in treating pain, inflammation and the like.

### Background art

Kits for preparing a medicament in the Pharmacy are known in the prior art. For instance, WO 2018/213932 A1 discloses a kit comprising cannabidiol (CBD) in an emulsion for preparing a medicament for treating conditions such as pain.

The use of the two-syringe technique for preparing an emulsion containing a medicament is disclosed in the prior art such as CN 2121950 U.

### Problem to be solved by the invention

The prior art does not disclose a kit for preparing a medicament containing an emulsion, wherein the kit contains components, equipment, and parts for carrying out an emulsification using the two-syringe technique. In particular, the prior art lacks disclosure of a kit not containing the active ingredient of the medicament.

Therefore, the problem underlying the present invention was to provide an improved kit for preparing a medicament containing an emulsion, wherein the kit allows an easy and simple preparation of the emulsion and the provision a customized medicament.

Another problem to be solved was the provision of an improved medicament containing cannabinoids in an emulsion in the form of a nasal spray, rectal suppository or vaginal suppository, and the provision of an improved method of preparing the same.

### Summary of the invention

The problems underlying the present invention were solved by providing the subject-matter defined in the following points [1] to [15].
[1] A kit for preparing a medicament, wherein the kit does not contain an active ingredient of the medicament and contains components, equipment, and parts
   for carrying out, after adding the active ingredient, a two-syringe emulsification to prepare an emulsion containing the active ingredient; and
   for preparing the medicament using the emulsion.
[1-1] In a preferred kit of point [1], the components for carrying out, after adding the active ingredient, the two-syringe emulsification comprise an excipient composition capable of forming an emulsion either with a water-based or water-miscible liquid or with an oil-based liquid. This kit is usable for an active ingredient contained in a liquid, e.g. in the form of a liquid extract.
[1-2] In a preferred kit of point [1], the components for carrying out, after adding the active ingredient, the two-syringe emulsification comprise an excipient composition and a liquid for containing (e.g. dissolving) the active ingredient, wherein the excipient composition and the liquid are capable of forming an emulsion. This kit is usable for an active ingredient that is e.g. in the form of powder or concentrate and is to be dissolved or diluted in the liquid for containing the active ingredient.
[1-3] A preferred kit of point [1], [1-1], or [1-2] does not contain an active ingredient that is only available on prescription. In other words, all active ingredients optionally contained in the kit are prescription-free.
[2] The kit according to point [1], [1-1], [1-2], or [1-3], comprising a first syringe and a second syringe, a connector for reversibly connecting the nozzles of the two syringes thereby enabling fluid communication between the two syringes, an excipient composition containing an emulsifier and being capable of forming an emulsion either with an oil-based liquid or with a water-based or water-miscible liquid, and a medicament container for accommodating an emulsion.
[2-1] In a preferred kit of point [2], the excipient composition is capable of forming an emulsion with an oil-based liquid.
[3] The kit according to point [2] or [2-1], wherein the medicament container is selected from a nasal spray bottle, a mold for rectal suppositories, or a mold for vaginal suppositories.
[4] The kit according to point [2], wherein the excipient composition is an water-based liquid and contains 3 to 14 % of a non-ionic ethoxylated vegetable oil as an emulsifier and the medicament container is a nasal spray bottle; or the excipient composition is based on a hydroxylated fat and contains 5 to 20 % of 2-hydroxypropyl-β-cyclodextrin as an emulsifier and the medicament container is a mold for rectal suppositories; or the excipient composition is based on a hydroxylated fat and contains 1 to 5 % of polyethylene glycol stearate as an emulsifier and the medicament container is a mold for vaginal suppositories.
[5] A method of preparing a medicament comprising step (i) of providing an active ingredient and step (ii) of using the kit as described in any one of points [1], [1-1] etc. to [4] to prepare the medicament containing the active ingredient in an emulsion.

In other words, point [5] is directed to the use of an active ingredient in a method of preparing a medicament, wherein the method comprises using the kit as described in any one of points [1], [1-1] etc. to [4] to prepare the medicament containing the active ingredient in an emulsion.
[5-1] In a preferred method or use, respectively, of point [5], the active ingredient is provided in a first liquid containing the active ingredient in step (i), and the kit contains, as a component for carrying out the two-syringe emulsification, a second liquid capable of forming an emulsion with the first liquid, wherein the emulsion is prepared by subjecting the first liquid and the second liquid to the two-syringe emulsification.
[5-2] In a preferred method or use, respectively, of point [5], the kit contains a first liquid and, as a component for carrying out the two-syringe emulsification, a second liquid capable of forming an emulsion with the first liquid, and step (ii) comprises adding the active ingredient to the first liquid and preparing the emulsion by subjecting the first liquid containing the active ingredient and the second liquid to the two-syringe emulsification.
[6] The method or use, respectively, according to point [5], [5-1], or [5-2], wherein the active ingredient is only available on prescription.
[7] The method or use, respectively, according to point [6], wherein the active ingredient is or contains a cannabinoid.
[7-1] In a preferred method or use, respectively, of point [7], the active ingredient is a cannabinoid contained in an oil-based cannabis extract.
[8] The method or use, respectively, according to any one of points [5], [5-1] etc. to [7-1], wherein the kit contains a first syringe, a second syringe, a connector, a medicament container, and a second liquid, step (i) is the following step (a) and comprises providing the active ingredient in a first liquid, and step (ii) comprises the following steps (b) to (g); or wherein the kit contains a first syringe, a second syringe, a connector, a medicament container, a first liquid, and a second liquid, step (i) is carried out prior to the following step (a) and comprises adding the active ingredient to the first liquid, and step (ii) comprises the following steps (b) to (g):
   (a) providing the first liquid containing an active ingredient, e.g. a cannabinoid-containing oil-based cannabis extract;
   (b) filling the first liquid into a first syringe;
   (c) providing the second liquid, wherein the second liquid is capable of forming an emulsion with the first liquid;
   (d) filling the second liquid in a second syringe;
   (e) using the connector to connect the nozzle of the first syringe with the nozzle of the second syringe to provide a fluid communication between the first and the second syringe;
   (f) repeatedly moving the plungers of the two syringes back and forth thereby mixing the first liquid with the second liquid and forming an emulsion containing the active ingredient; and
   (g) filling the emulsion containing the active ingredient into the medicament container.
[9] A method of preparing a medicament containing a cannabinoid as an active ingredient, wherein the method comprises:
   (a) providing a first liquid containing the cannabinoid;
   (b) filling the first liquid into a first syringe;
   (c) providing a second liquid capable of forming an emulsion with the first liquid;
   (d) filling the second liquid in a second syringe;
   (e) connecting the nozzle of the first syringe with the nozzle of the second syringe to provide a fluid communication between the first and the second syringe;
   (f) repeatedly moving the plungers of the two syringes back and forth thereby mixing the first liquid with the second liquid and forming an emulsion containing the cannabinoid; and
   (g) filling the emulsion containing the cannabinoid into a medicament container.
[10] The method according to any one of points [5] to [9], wherein the medicament contains 2 to 8 % of non-ionic ethoxylated vegetable oil comprised as an emulsifier in the second liquid and is in the form of a nasal spray, or the medicament contains 5 to 20 % of 2-hydroxypropyl-β-cyclodextrin comprised as an emulsifier in the second liquid and is in the form of a rectal suppository, or the medicament contains 1 to 5 % of polyethylene glycol stearate comprised as an emulsifier in the second liquid and is in the form of a vaginal suppository.
[11] A kit for preparing a medicament containing a cannabinoid, wherein (i) the kit does not contain the cannabinoid and contains components, equipment and parts for carrying out, after adding the cannabinoid, a two-syringe emulsification to prepare the medicament containing the cannabinoid; or (ii) the kit contains the cannabinoid and components, equipment and parts for carrying out a two-syringe emulsification to prepare the medicament containing the cannabinoid.
[11-1] In preferred embodiment of point [11], the cannabinoid is contained in an oil-based cannabis extract. Hence, the kit of point [11-1](i) contains components, equipment and parts for carrying out, after adding a cannabinoid-containing oil-based cannabis extract, a two-syringe emulsification to prepare an emulsion containing the cannabinoid; or the kit of [11 - 1](ii) contains a cannabinoid-containing oil-based cannabis extract and components, equipment and parts for carrying out a two-syringe emulsification to prepare an emulsion containing cannabinoid.
[11-2] Preferably, the kit of point [11] or [11-1] contains a medicament container selected from a nasal spray bottle, a mold for rectal suppositories, or a mold for vaginal suppositories.
[12] The use of a cannabinoid in a method of preparing a medicament containing the cannabinoid by carrying out a two-syringe emulsification.
[12-1] Preferably, the use of a cannabinoid of point [12] is the use of a cannabinoid-containing oil-based cannabis extract.
[12-2] In a preferred embodiment of point [12] or [12-1], the method of preparing a medicament containing the cannabinoid is the method of point [9].
[12-3] In a preferred embodiment of point [12], [12-1], or [12-3], the method of preparing a medicament containing the cannabinoid comprises employing the kit of point [11], [11-1], or [11-2].
[12-4] A preferred embodiment of the use of point [12] is the use of a cannabinoid-containing oil-based cannabis extract in a method of preparing a medicament of point [9] by using the kit of point [11-1], preferably [11-1](i).
[13] A medicament obtainable by the method of any of points [5] to [9].
[13-1] In a preferred embodiment, the medicament of point [13] is suitable for use in mucosal or transdermal administration.
[14] A medicament containing a cannabinoid in an emulsion, wherein the medicament contains 2 to 8 % of non-ionic ethoxylated vegetable oil as an emulsifier and is in the form of a nasal spray, or the medicament contains 5 to 20 % of 2-hydroxypropyl-β-cyclodextrin as an emulsifier and is in the form of a rectal suppository, or the medicament contains 1 to 5 % of polyethylene glycol stearate as an emulsifier and is in the form of a vaginal suppository.
[15] A medicament according to point [13] or [14] in the form of a nasal spray for use in treating pain, inflammation, Tourette syndrome, epilepsy, schizophrenia, anxiety or panic attacks, spasticity, neurodegenerative disorders, migraines, anorexia, nausea or sleep disturbances; or in the form of rectal suppository for use in treating chronic pain, anxiety or insomnia; or in the form of a vaginal suppository for use in treating endometriosis pain, menstrual related pain and cramps, dysmenorrhoea, premenstrual syndrome, sexual pain disorders, vaginismus, vulvodynia, dyspareunia, ovarian or cervical cancer pain, pelvic inflammatory disease, bacterial vaginosis, infections and inflammations.

### Advantages of the invention

The kits according to the present invention are compounding kits for pharmacists and enable a simple and safe preparation of a medicament containing an emulsion.

Since the kit does not contain the active ingredient, which may be a prescription drug, the kit is available without any restrictions.

The preparation of the medicament may be customized at the stage of adding the active ingredient to the components of the kit.

In combination with cannabis extracts, the kits enable a range of new administrational options for medical cannabis, while still being classified as preparatory drugs under German regulations. The kits bring new therapy options. Examples of kits may contain the components necessary for preparing a nasal spray, vaginal and rectal suppository, liquid transdermal patch, and eye tincture.

### Embodiments of the invention

The first aspect of the present invention relates to a kit for preparing a medicament, wherein the kit does not contain an active ingredient of the medicament and contains components, equipment, and parts for carrying out, after adding the active ingredient, a two-syringe emulsification to prepare an emulsion containing the active ingredient; and for preparing the medicament using the emulsion.

The second aspect of the present invention relates to a method of preparing a medicament comprising step (i) of providing an active ingredient and step (ii) of using the kit of the first aspect and carrying out two-syringe emulsification to prepare the medicament containing the active ingredient in an emulsion.

The third aspect of the present invention relates to a method of preparing a medicament containing a cannabinoid as an active ingredient, wherein the method comprises carrying out two-syringe emulsification to prepare the medicament containing cannabidiol in an emulsion. The third aspect is independent of using the kit of the first aspect.

The fourth aspect of the present invention relates to a kit for preparing a medicament containing a cannabinoid, wherein the kit (i) may or (ii) may not contain the cannabinoid, wherein the kit contains components, equipment and parts for carrying out a two-syringe emulsification to prepare the medicament containing the cannabinoid.

The fifth aspect of the present invention relates to the use of a cannabinoid in a method of preparing a medicament containing the cannabinoid by carrying out a two-syringe emulsification.

The sixth aspect of the present invention relates to a medicament obtainable by the method of the second or third aspect.

The seventh aspect of the present invention relates to a medicament containing a cannabinoid in an emulsion, wherein the medicament contains 2 to 8 mass % of non-ionic ethoxylated vegetable oil as an emulsifier and is in the form of a nasal spray, or the medicament contains 5 to 20 mass % of 2-hydroxypropyl-β-cyclodextrin as an emulsifier and is in the form of a rectal suppository, or the medicament contains 1 to 5 mass % of polyethylene glycol stearate as an emulsifier and is in the form of a vaginal suppository.

The eighth aspect of the present invention relates to specific medical uses of the medicament of the sixth aspect or the seventh aspect.

As used herein, the singular forms "a," "an," and "the" may be intended to include the plural forms as well, unless the context clearly indicates otherwise. For instance, the term "an active ingredient" means one or more active ingredients, e.g. one or more cannabinoids, or "a" component or "containing a" component means one or more components. Such singular form formulations are generally meant to include more than one of the indicated component, unless indicated otherwise.

The terms "comprises," "comprising," "including," and "having," are inclusive and therefore specify the presence of stated features, steps, and/or components, but do not preclude the presence or addition of one or more other features, steps, components and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

The terms "first" and "second" are used herein to describe liquids or syringes differing from one another and do not imply a sequence or order unless clearly indicated by the context. Thus, a first liquid could be termed a second liquid and vice versa, and a first syringe could be termed a second syringe and vice versa.

In the present invention, percentages (%) indicate the mass fraction and are percentages by mass (% by mass) unless explicitly indicated otherwise.

A "subject" according to some embodiments is an individual whose signs and symptoms, physical exams findings and/or psychological exam findings are to be determined and recorded in conjunction with the individual's condition (i.e., disease or disorder status) and/or response to a candidate drug or treatment. A subject according to some embodiments of the present invention includes a patient in need of therapeutic treatment for a disorder treatable with the medicament. "Treatment," as used herein, includes the attempt to effect a change in a particular aspect of a subject's health (i.e., directed to a particular disease, disorder, or condition) or alleviate or mitigate symptoms of a particular disease, disorder or condition. "Alleviate" as used herein, is meant to include complete elimination as well as any clinically or quantitatively measurable reduction in the subject's symptoms and/or discomfort.

The term "pharmaceutically acceptable" means that the compound or combination of compounds is compatible with the remaining ingredients of the formulation for pharmaceutical use, and that it is generally safe for administering to humans according to established governmental standards.

The term "a therapeutically effective amount" means an amount of an active ingredient sufficient to induce a therapeutic or prophylactic effect in treating or to alleviating or mitigating symptoms associated with the diseases or disorders mentioned herein.

### Kit

The kit according to the present invention is a kit of the first aspect containing components, equipment and parts for carrying out, after adding the active ingredient not being part of the kit, a two-syringe emulsification to prepare an emulsion containing the active ingredient; and for preparing the medicament using the emulsion; or a kit of the fourth aspect, which may or may not contain the active ingredient The following description relates to both the first and fourth aspect, if not stated otherwise.

The components contained in the kit may be chemical compounds, substances, or solvents. A component may be an emulsifier, wetting agent, a preservative, water, or an excipient composition, e.g. the second liquid used herein.

The equipment contained in the kit may comprise the means necessary for carrying out the preparation of the medicament but not being part of the final medicament, e.g., two syringes and a connector for connecting the nozzles of the syringes.

The parts contained in the kit may comprise the medicament container such as a nasal spray bottle or suppository molds.

The two-syringe emulsification is a method of preparing an emulsion and comprises providing a first syringe containing a first liquid; providing a second syringe containing a second liquid, the second liquid being capable of forming an emulsion with the first liquid; connecting the nozzle of the first syringe with the nozzle of the second syringe to provide a fluid communication between the first and the second syringe; repeatedly moving the plungers of the two syringes back and forth thereby mixing the first liquid with the second liquid and forming an emulsion.

The active ingredient is added to the components contained in the kit for preparing a composition to be subjected to the two-syringe emulsification. The components contained in the kit may be the excipient composition described herein.

The components for carrying out, after adding the active ingredient, the two-syringe emulsification may comprise an excipient composition capable of forming an emulsion either with a water-based or water-miscible liquid or with an oil-based liquid. The excipient composition may be an oil-based liquid to form an emulsion with a water-based or water-miscible liquid. The excipient composition may be a water-based or water-miscible liquid, e.g., an aqueous liquid or a hydrophilic substance, to form an emulsion with an oil-based liquid. The hydrophilic substance may be a medium-hydroxyl hard fat that is solid at room temperature and melted at elevated temperatures to become a liquid that forms an emulsion with an oil-based liquid. The emulsion may solidify upon cooling to room temperature to form a solid emulsion usable as a suppository.

According to the first aspect, the kit does not contain an active ingredient of the medicament. This means that the kit does not contain one or more active ingredients necessary for the medicament to serve its desired function for treating a condition. In one embodiment, the kit does not contain an active ingredient that is only available on prescription (prescription drug) such as medical cannabis extracts; however, it may contain prescription-free active ingredients. Since the kit is used for preparing a medicament, the preparation of the medicament is preferably carried out in the pharmacy, i.e. by a qualified person.

The preparation of the medicament requires the active ingredient and other elements such as components, equipment and parts. The kit does not necessarily contain all the other elements required for preparing the medicament. Typically, the kit may not contain standard pharmacy equipment or components such a beaker, analytical balance, steel spoons, glass rod, cleaning paper, water, water bath, and drying oven. However, the kit may contain the other elements required for preparing the medicament.

The kit according to the present invention contains equipment for carrying out a two-syringe emulsification to prepare an emulsion. To that end, the kit may contain a first syringe, a second syringe, and a connector for reversibly connecting the nozzles of the two syringes thereby enabling fluid communication between the two syringes. This equipment may also be used in the methods of preparing a medicament according to the present invention to carry out the two-syringe emulsification. The means for reversibly connecting the syringes may be a luer lock system exemplified below.

A syringe is a simple reciprocating pump consisting of a plunger (or a piston) that fits tightly within a cylindrical tube. The plunger can be linearly pulled and pushed along the inside of the tube, allowing the syringe to take in and expel fluid, i.e. liquid or gas, through a nozzle, i.e. a discharge orifice, at the front open end of the tube. The syringe contains a needle adapter such as a luer lock fitting at the front end of the tube.

The kit contains a connector for reversibly connecting the nozzle of the first syringe and the nozzle of the second syringe thereby enabling fluid communication between the two syringes. The connector may consist of a single part or several parts. A typical connector system is based on a luer taper also refer to as luer lock. The luer lock connector can connect the two syringes each equipped with a luer lock fitting.

Examples of kits may contain the components, equipment and parts necessary for preparing a nasal spray, vaginal and rectal suppository, liquid transdermal patch, and eye tincture.

In the case of a nasal spray, the kit may contain the following: 3 units nasal spray bottle, 3 units spray-lid, 2 units of 50 ml Luer-Lock syringe, 1 unit of 20 ml Luer-Lock syringe, 2 units Luer-Lock inter-connector, 1 unit Luer-Lock cap, 1 unit Luer-Lock female connector, 1 transport box, 1 manufacturing protocol, plausibility and identification documents for pharmacists, the excipient composition described herein, and optionally 1 brochure with dosing recommendations and product card.

In the case of a rectal or a vaginal suppository, the kit may contain the following: 1 unit with 10 rectal suppository forms or vaginal suppository forms, respectively, including a stripe of closing tap, 2 units 35 ml Luer-Lock syringes, 1 unit 10 ml Luer-Lock syringe, 2 units Luer-Lock inter-connector, 1 unit Luer-Lock female connector, 1 transport box, 1 manufacturing protocol, plausibility and identification documents for pharmacists, the excipient composition described herein, and optionally 1 brochure with dosing recommendations and a product card.

### Active ingredient

In the present application, the active ingredient to be added to the kit of the present invention, the active ingredient such as a medical cannabis extract containing THC and/or CBD used to prepare the medicament of the present invention, and the active ingredient contained in the medicament of the present invention are collectively referred to as "the active ingredient", unless stated otherwise. In other words, the term "the active ingredient" refers to the first to eighth aspect and to all embodiments of the present invention, unless stated otherwise.

The active ingredient is a pharmaceutically active ingredient contained in the medicament. The active ingredient is not limited to any specific disease or disorder to be treated. The only restriction is that the active ingredient is contained in an emulsion or may be provided in the form of an emulsion. The advantages of the present invention apply to all kinds of active ingredients, as long as they can be subjected to a two-syringe emulsification to obtain an emulsion.

In one embodiment, the active ingredient is only available on prescription. An example of an active ingredient is THC.

The method of the second and third aspect comprises a step (a) of providing a first liquid containing an active ingredient such as a cannabinoid. The active ingredient may be added to the first liquid, or the active ingredient may be a component of the first liquid, e.g. as a component of an extract of a plant. Hence, the step of providing a first liquid containing an active ingredient may be a step of providing a plant extract containing the active ingredient. In the case of THC, the plant extract may be an oil-based extract of a cannabis plant.

Cannabinoids, the active constituents of cannabis, are soluble in highly non-polar solvents (i.e. in substances such as chloroform, dichloromethane and high concentrations of alcohol); they also have limited solubility in glycols. Tetrahydrocannabinol (THC) is an oily liquid at room temperature; CBD is an oil soluble solid. Both have very low solubility in aqueous excipients.

Cannabis sativa (i.e., cannabis) is a hemp plant that grows throughout temperate and tropical climates. The leaves and flowering tops of Cannabis plants contain more than 70 different phytocannabinoids. The principal cannabinoids are delta-9-tetrahydrocannabinol (THC), cannabinol (CBN), and cannabidiol (CBD). CBD accounts for up to 40 % of the plant's extract.

CBD lacks detectable psychoactivity and may thus be a preferred active ingredient. In other embodiments, THC may be preferred. THC and CBD may be derived synthetically or may be obtained by extraction from a natural source such as a cultivar of cannabis sativa.

The active ingredient such as CBD or THC may be extracted by using a lipophilic solvent such as oils. The oil may be selected from the group consisting of a pharmaceutically acceptable vegetable oil, a fatty acid, a monoglyceride, a diglyceride, sucrose acetate isobutyrate, a synthetic triglyceride, and a combination thereof.

### Medicament

In the present application, the medicament that can be prepared by using the kit of the present invention, the medicament that is prepared by the method of the present invention, and the medicament of the present invention are collectively referred to as "the medicament", unless stated otherwise. In other words, the term "the medicament" refers to the first to eighth aspect and to all embodiments of the present invention, unless stated otherwise.

The medicament is a pharmaceutical composition in the form of an emulsion. Being based on an emulsion, the medicament is particularly suitable for mucosal administration such as administration to the mucosa of the nose, mouth, vagina or rectum, or for transdermal administration. The emulsion is preferably in the formulation format of a nasal spray, a rectal suppository, a vaginal suppository, a liquid transdermal patch, or eye tincture.

The medicaments of the present invention are emulsions that are formulated with therapeutically effective amounts of active ingredient such as a cannabinoid and are administered to treat disorders or disease states or alleviate or mitigate symptoms thereof that are treatable with the active ingredient such as a cannabinoid.

In addition to the active ingredient mentioned herein, the medicament of the present invention may contain other active ingredients. Moreover, the medicament may contain components other than pharmaceutically active ingredients. In other words, the medicament may be a composition containing the active ingredient mentioned herein and non-pharmaceutical components. The medicament or composition, respectively, may be employed for non-medical uses such as cosmetic uses. However, the medical uses are preferred.

In one embodiment, the medicament according to the present invention contains a cannabinoid as an active ingredient. Other active ingredients are not excluded. In particular, the medicament may contain other compounds of Cannabis sativa.

The present invention provides a medicament containing an emulsion formulated with a cannabinoid in amounts ranging from between about 0.1 % to about 25 % or more by weight, for administration to deliver a therapeutically effective amount of a cannabinoid to effectively treat disorders or disease states treatable with the cannabinoid, or to alleviate or mitigate symptoms associated therewith.

In one embodiment, the medicament does not contain a high amount or does not contain any amount of components having psychoactivity. Such a medicament may contain CBD. As to CBD purity, the cannabinoid utilized to formulate the medicament of the present invention has a CBD purity of preferably about 50 %, more preferably a CBD purity of about 60 %, more preferably a CBD purity of about 70 %, more preferably a CBD purity of about 80 %, even more preferably a CBD purity of about 90 %, about 95 %, and about 98 %, and about 99 %, and even more preferably a CBD purity of about 99 %, and most preferably a CBD purity of about 100 %. In other words, the mass ratio of CBD to THC in the medicament may be at least 10, preferably at least 20, more preferably at least 50.

In one embodiment, the medicament such as a THC-containing medicament may be in the form of a nasal spray. In other words, the medicament is usable as a nasal spray. The nasal spray may be contained in a nasal spray bottle suitable for administering the nasal spray into the nose of a subject.

The nasal spray may be used by patients who require a rapid therapeutic onset of medical cannabis, e.g. for use in pain peak control and management of sudden cramps, chronic ticks in neurodegenerative diseases or sudden strong states of exhaustion connected to burnout, panic attacks or post-traumatic-stress-disorders. With the nasal spray application, patients in these indication areas regain the security of participating in daily life. The spray combines rapid onset with the convenience of use, particularly in public, without the smell or visibility of vaping. It goes hand-in-hand with patients' needs in everyday life. Key target indications include pain and inflammation, Tourette syndrome, epilepsy, schizophrenia, anxiety/panic attacks, spasticity, neurodegenerative disorders, migraines, cancer/HIV associated symptoms such as anorexia (loss of appetite), nausea or sleep disturbances.

In one embodiment, the medicament such as a THC-containing medicament may be in the form of a rectal suppository. In other words, the medicament is usable as a rectal suppository. The rectal suppository may be the medicament as such or may contain the medicament in a container or coating suitable for being administered into the rectum of a subject. The kit according to the present invention supports a standardized and swift compounding of rectal suppositories in the Pharmacy.

For instance, a rectal application of THC offers broader therapy options in all established cannabis indications as an alternative to vaping or oral application, particularly in chronic therapy areas such as in chronic pain management, palliative care or geriatric care. The rectal suppository can be used as an alternative application option in all standard indications for medical cannabis, particularly in chronic therapies such as chronic pain management, palliative care or geriatric care.

The excipient composition for a rectal suppository may contain Witepsol W25 (a medium-hydroxyl hard fat base), HPβCD (2-Hydroxypropyl-beta-cyclodestrin; to improve the solubility and bioavailability of lipophilic substances in aqueous solutions), and bleached wax (to enhance consistency). The excipient composition may contain 5 to 20 % of HPβCD. The ideal molar mass ratio of HPβCD to cannabinoid is 1:1, corresponding to 17 %. In the Examples of the present invention, the content was 12 %, which is in accordance with a maximum concentration of 12 % recommended by EMA guidelines.

In one embodiment, the medicament such as a THC-containing medicament may be in the form of a vaginal suppository. In other words, the medicament is usable as a vaginal suppository. The vaginal suppository may be the medicament as such or may contain the medicament in a container suitable for being administered into the vagina of a subject. The kit according to the present invention supports a standardized and swift compounding of vaginal suppositories in the Pharmacy.

For instance, a vaginal application of a cannabinoid offers broader therapy options for women in all established cannabis indications as an alternative to vaping or oral application, such as chronic pain, palliative care or oncology care. Furthermore, the vaginal suppositories have the potential to open new indication areas in women's health as well, such as endometriosis pain, menopause discomfort or other periodic pain, as well as vaginal infections and inflammations. With this, the vaginal suppository kit will alleviate unnecessary suffering, and improve the vaginal health and quality of life of women. Target indications are all standard indications for medical cannabis. New options include the use to treat or alleviate endometriosis pain, menstrual related pain and cramps, dysmenorrhoea (painful periods), premenstrual syndrome (PMS), sexual pain disorders, vaginismus (vaginal cramp), vulvodynia (pain in genital area), dyspareunia (painful intercourse), ovarian/cervical cancer pain. Further indications include pelvic inflammatory disease (PID), bacterial vaginosis, infections, and inflammations, strengthen the overall health of the vagina.

The excipient composition for a vaginal suppository may contain Witepsol W25, bleached wax, and Macrogol-8-stearate. The excipient composition may contain 0.5 to 5 % of HPβCD. The ideal molar mass ratio of HPβCD to cannabinoid is 1:1, corresponding to 17 %. In the Examples of the present invention, the content was 12 %, which is in accordance with a maximum concentration of 12 % recommended by EMA guidelines.

### Emulsion

In the present application, the emulsion to be prepared by using the kit of the present invention, the emulsion prepared by two-syringe emulsification in the preparation of a medicament, and the emulsion contained in the medicament of the present invention are collectively referred to as "the emulsion", unless stated otherwise. In other words, the term "the emulsion" refers to the first to eighth aspect and to all embodiments of the present invention, unless stated otherwise.

An emulsion is a mixture of two or more liquids that are immiscible owing to liquid-liquid phase separation. The emulsion may be an "oil-in-water" emulsion, in which a oil-based liquid is the dispersed phase, and a water-based or water-miscible liquid is the continuous phase, or a "water-in-oil" emulsion, in which a water-based or water-miscible liquid is the dispersed phase and oil-based liquid is the continuous phase.

In the present invention, an "oil" is a lipid, i.e. an organic molecule soluble in nonpolar solvents. The oil may be selected from saturated or unsaturated C₆-C₂₄ fatty acids, mono-, di-, or triesters of C₄-C₂₄ carboxylic acids with glycerol, and combinations thereof. Preferably, the oil in the oil-based liquid is one or more pharmaceutically acceptable oil. The oil may be selected from the group consisting of a pharmaceutically acceptable vegetable oil, a fatty acid, a monoglyceride, a diglyceride, a triglyceride, and a combination thereof.

The water-based liquid is an aqueous liquid. The water-miscible is a non-aqueous and at least partly hydrophilic substance such as a solid triglyceride modified with hydroxyl groups. For instance, the at least partly hydrophilic substance is a medium-hydroxyl hard fat that is solid at room temperature and melted at elevated temperatures (e.g., 40 °C) to become a liquid that is water-miscible and oil-immiscible.

An oil-based liquid contains at least 50 % of at least one oil, a water-based liquid contains at least 50 % of water, and a water-miscible liquid contains at least 50 % of a non-aqueous and at least partly hydrophilic substance. Preferably, each of the water-based liquid, the water-miscible liquid, and the oil-based liquid is a solution, hence no suspension or emulsion.

Herein, the term "emulsion" covers a liquid, semi-solid and solid emulsions. An example of a liquid emulsion is a nasal spray. Examples of semi-solid emulsions or solid emulsions, respectively, are rectal and vaginal suppositories. In the case of a semi-solid or solid emulsion, the emulsion as such may be the suppository or the emulsion may be contained in a medicament container such as a capsule suitable for being administered to the rectum or vagina, respectively. Semi-solid emulsions and solid emulsions form liquid emulsions after melting; for instance, they are solid at up to 25 °C (e.g., at room temperature of 21 °C) and melt at more than 30 °C (e.g., at body temperature of 37 °C).

The emulsion used in the present invention is formed by the first liquid and the second liquid. The first liquid and the second liquid do not form a solution at temperatures of 10 to 40 °C. Preferably, the first liquid is oil-based and the second liquid is water-based or water-miscible. In the case of a nasal spray, the emulsion preferably contains 10 to 90 % of an oil-based first liquid and 90 to 10 % of a water-based second liquid. In the case of a rectal suppository, the emulsion preferably contains 10 to 90 % of an oil-based first liquid and 90 to 10 % of a semi-solid or solid excipient composition, which may be melted to give a water-miscible second liquid.

For emulsification of oil into water or vice versa any single or combination of surfactants can be used. Examples of surfactants include any one or mixture of surfactants. The surfactants may belong to non-ionic, anionic or cationic surfactants: Glycol Distearate, Sorbitan Trioleate, Propylene Glycol Isostearate, Glycol Stearate, Sorbitan Sesquioleate, Lecithin, Sorbitan Oleate, Sorbitan Monostearate NF, Sorbitan Stearate, Sorbitan Isostearate, Steareth-2, Oleth-2, Glyceryl Laurate, Ceteth-2, PEG-30 Dipolyhydroxystearate, Glyceryl Stearate SE, Sorbitan Stearate (and) Sucrose Cocoate, PEG-4 Dilaurate, Methyl Glucose Sesquistearate, Lecithin HLB (variable) PEG-8 Dioleate, Sorbitan Laurate, Sorbitan Laurate , PEG-40 Sorbitan Peroleate, a polyoxyl glyceride, such as Labrafil^{®} M1944CS, Laureth-4, PEG-7 Glyceryl Cocoate, PEG-20 Almond Glycerides, PEG-25 Hydrogenated Castor Oil, Stearamide MEA, Glyceryl Stearate (and) PEG- 100 Stearate, Polysorbate 85, PEG-7 Olivate, Cetearyl Glucoside, Stearamide MEA, PEG-8 Oleate, Polyglyceryl-3 Methyglucose Distearate, Oleth- 10, Oleth- 10/ Polyoxyl 10 Oleyl Ether NF, Ceteth-10, PEG-8 Laurate, Cocamide MEA, Polysorbate 60 NF, Polysorbate 60, Polysorbate 80, Isosteareth-20, PEG-60 Almond Glycerides, PEG-20 Methyl Glucose Sesquistearate, Ceteareth-20, Oleth- 20, Steareth-20, Steareth-20 , Steareth-21, Steareth-21 , Ceteth-20, Steareth- 100.

Emulsifiers may be used either alone or in combination. If two or more emulsifiers are to be combined, they may be mixed at any desired proportions. For instance, the emulsion may contain sucrose stearate, sucrose cocoate, sucrose distearate, mineral oil, propylene glycol, 2-ethyl- 1 ,3- hexanediol, polyoxypropylene-15-stearyl ether, water, or combinations thereof.

The content of the emulsifier in the medicament should be such that stable emulsification will be assured during manufacture and for that matter it is preferably 0.05 - 10 %, more preferably 0.05 - 5 %, of the total quantity of the medicament.

The emulsion can be formulated in a variety of formulation formats such as cream, lotion, liquid, gel, foam, drops, suppository, ointments, shampoo, soap bar, sprays and patches.

Other components may be contained so long as they do not interfere significantly with the stability of the emulsified active ingredient, e.g. THC. Suitable liquid or gel-based components may include water, physiological salt solutions, alcohols (e.g., methanol, ethanol, propanol, or butanol), glycerol, glycols (e.g., ethylene glycol, propylene glycol, or ethoxy diglycol), polyethylene glycol (e.g., MW 400 to 20,000), water-alcohol/glycol blends, and the like. Suitable components further include aqueous and oleaginous carriers such as, for example, white petrolatum, isopropyl myristate, lanolin or lanolin alcohols, mineral oil, fragrant or essential oil, sorbitan mono-oleate, cetostearyl alcohol (together or in various combinations), and detergents (e.g., polysorbates (Tweens) such as polysorbate 20, 40, 60, or 80; polyoxyl stearate; or sodium lauryl sulfate). One or more components can be mixed with water to form a lotion, gel, cream, semi-solid composition, or the like.

Preservatives may also be included, such as one or more of butylparaben, methylparaben, propylparaben, benzyl alcohol, and ethylene diamine tetraacetate salts.

### Excipient composition

The excipient composition may be contained in the kit of the first and fourth aspect of the present invention. The excipient composition may be the components of the kit for carrying out the two-syringe emulsification. The excipient composition optionally contains an emulsifier and is capable of forming an emulsion with an oil-based liquid. The excipient composition may be liquid or solid. Preferably, the excipient composition is a water-based or water-miscible liquid or a solid that is water-miscible after melting. In the case of a liquid, it may be used as the second liquid in the methods of the present invention. In the case of a solid, it may be melted to give the second liquid. The melting temperature may be 30 °C or higher.

The components of the kit for carrying out the two-syringe emulsification may be an excipient composition to be used as the second liquid. In that case, the first liquid is capable of forming an emulsion with the second liquid. Alternatively, the components of the kit for carrying out the two-syringe emulsification may comprise an excipient composition to be used as the second liquid and an additional liquid capable of forming an emulsion with the second liquid. In that case, the active ingredient may be added to the additional liquid to give the first liquid. Preferably, the second liquid and the additional liquid are contained in the kit as separate components.

The excipient composition may include, but is not limited to solvents, dispersion media, coatings, antibacterial agents, antifungal agents, isotonic and/or absorption delaying agents and the like.

### Nasal spray

In the present application, the nasal spray obtainable by using of the kit of the present invention, the nasal spray obtainable by carrying out the method of preparing a medicament such as a THC-containing medicament of the present invention, and the nasal spray being the form of the medicament of the present invention are collectively referred to as "the nasal spray", unless stated otherwise. In other words, the term "the nasal spray" refers to the first to eighth aspect and to all embodiments of the present invention, unless stated otherwise.

The medicament may be in form of a nasal spray. The nasal spray is used for topical application into the nasal cavity of a subject. The nasal spray is a system for dispensing intranasally a precise dosage amount of a mentioned according to the present invention at an optimal anatomical location within each nostril of the subject, so that an effective amount of the medicament such as THC is deposited within each nostril at the optimal anatomical location, i.e., the nasal vestibule, to use the nasal spray to effectively treat subjects to treat disease states or alleviate or mitigate symptoms thereof treatable with the medicament such as THC.

The excipient composition for a nasal spray may contain preserved water (i.e. purified water with 0.075 % of methyl 4-hydroxybenzoate and 0.025 % of propyl 4-hydroxybenzoate), Kolliphor^{®} ELP (is a non-ionic emulsifier made by reacting ethylene oxide with castor oil in a ratio of 35:1 (Polyoxyl-35-castor oil)), glycerol (a wetting agent that enhances nose feeling), xanthan gum (a consistency factor for water phase), and a carboxymethyl cellulose (having a viscosity of e.g. 600 mPa; makes the spray stickier, reduces dropping effects).

The excipient composition may be based on a conventional excipient composition used for nasal spray containing an emulsion. In the case of a cannabinoid as an active ingredient, the excipient composition preferably additionally contains 2 to 8 % of a non-ionic ethoxylated vegetable oil such as Kolliphor ELP.

### Suppository

In the present application, the suppository obtainable by using of the kit of the present invention, the suppository obtainable by carrying out the method of preparing a medicament such as a THC-containing medicament of the present invention, and the suppository being the form of the medicament of the present invention are collectively referred to as "the suppository", unless stated otherwise. In other words, the term "the suppository" (rectal suppository or vaginal suppository) refers to the first to eighth aspect and to all embodiments of the present invention, unless stated otherwise.

The medicament may be in form of a suppository such as a rectal suppository or a vaginal suppository. Preferably, the suppository is semi-solid or solid. The suppository has a preferred melting temperature of 34 - 37°C. When administered, the suppository melts at body temperature to release the active ingredient.

In the method of preparing a suppository, the first and the second liquid are each kept at a temperature suitable for emulsification, which is preferably 35 - 60°C, more preferably 35 - 45°C. Since the suppository may be semi-solid or solid at room temperature due to the content of components such as hard fat, it may be necessary to elevate the temperature, e.g. to 50 - 80°C to melt the components. After preparing the emulsion and molding, the suppository may be solidified at room temperature and may be removed from the mold. The semi-solid or solid suppository may be based on oils and fats such as hard fat or cocoa butter, hardened vegetable oils and, especially, mixtures of mono-, di- and triglycerides of saturated fatty acids having 10 to 18 carbon atoms. Glycerol fatty acid esters (mono-, di- and triglycerides) may be preferred. In particular, hard fat which is a semi-synthetic triglyceride may be preferred. The hard fat is mainly composed of a triglyceride of saturated fatty acids. The hard fat may be obtained by hydrolyzing coconut oil, palm kernel oil, etc., adjusting the length of the carbon chain in the obtained fatty acid or hydrogenating the same to yield the desired glycerin ester of the fatty acid. The thus obtained glycerin ester of fatty acids has the fatty acids with carbon chain length of 6 - 30 and it is primarily an ester with glycerol which is triglyceride, occasionally containing small amounts of unsaturated bonds or mono- and di-glycerides. The physical properties of the hard fat can be suitably changed by altering the degree of esterification, carbon chain length, the number of unsaturated bonds, etc. or by adding a suitable additive. Examples of the hard fat that can be used in the present invention include Witepsol, i.e. hard fats with hydroxyl values between 20-50 comprising a mixture of 65-80% of triglycerides, 10-35% of diglycerides and 1-5 % of monoglycerides. The amount of partial glycerides promotes wetting of mucous membranes. An example is Witepsol W25, a medium-hydroxyl hard fat base having a hydroxyl value of (modifying KOH/g) of 20-30 and a melting point of about 34 °C.

The emulsifier to be used in the invention may be selected from among the nonionic or ionic surfactants. Exemplary nonionic surfactants include sorbitan fatty acid esters, glycerol fatty acid esters, glycerol, decaglycerin fatty acid esters, polyglycerin fatty acid esters, propylene glycol fatty acid esters, pentaerythritol fatty acid esters, polyoxyethylene sorbitan fatty acid esters, polyoxyethylene sorbitol fatty acid esters, polyoxyethylene glycerin fatty acid esters, polyethylene glycol fatty acid esters, sucrose fatty acid esters, polyoxyethylene alkyl ethers, polyoxyethylene phytosterols, polyoxyethylene phytostanols, polyoxyethylene polyoxypropylene glycol, polyoxyethylene castor oil, polyoxyethylene hydrogenated castor oil, polyoxyethylene lanolin, polyoxyethylene lanolin alcohol, beeswax derivatives, polyoxyethylene alkylamines and polyoxyethylene fatty acid amides. Exemplary anionic surfactants include alkyl sulfates, polyoxyethylene alkyl ether sulfates, N-acylamino acid or salts thereof, and polyoxyethylene alkyl ether phosphates. Exemplary cationic surfactants include alkyl ammonium salts and alkylbenzyl ammonium salts. Exemplary amphoteric surfactants include betaine acetate and lecithin.

An emulsifier having high affinity for the hard fat may be preferred. It is also preferred to use an emulsifier that allows the aqueous-phase component to be emulsified stably within the hard fat having high practical value for use with suppositories and which, after solidifying, either retains a high hardness enough to withstand use as suppository or assures uniform and satisfactory properties as a pharmaceutical preparation.

From practical viewpoints, i.e., low irritation upon use as a pharmaceutical, the ability to guarantee stable emulsification during manufacture and the added advantage of good shaping upon solidification, it may be preferred to use at least one emulsifier selected from the group consisting of sucrose fatty acid esters, glycerol, polyoxyethylene polyoxypropylene glycol and polyoxyethylene hydrogenated castor oil.

The emulsion is prepared by using the two-syringe emulsification method. Stable emulsification may be evidenced by, for example, the small diameter of the particles in the emulsion, as well as the absence of separation of the aqueous phase, aggregation and sedimentation in the emulsified state during melting or filling into suppository containers. Such stability in emulsification and shaping property upon solidification need be fully taken into account when selecting a suitable emulsifier.

### Examples

The following abbreviations and components are used:
NRF: "Neue Rezeptur-Formularium" (English: "New Prescription Formulatory"), formulatory laying down standards for compounding medicaments;
q. s.: quantum satis;
Preserved water: purified water with 0.075 % methyl 4-hydroxybenzoate and 0.025 %propyl 4-hydroxybenzoate;
Kolliphor^{®} ELP: hot emulsifier Pharma grade;
Glycerol: wetting agent, enhances nose feeling;
Xanthan gum: consistency factor for water phase;
Carboxymethyl cellulose (viscosity 600m Pa): makes spray stickier, reduces dropping effects; HPβCD: 2-Hydroxypropil-beta-cyclodestrin: to improve solubility and bioavailability, ideal molar mass ratio of HPβCD to cannabinoid is 1:1, corresponding to 17 %. In the Examples, the content was 12 %, which is in accordance with a maximum concentration of 12 % recommended by EMA guidelines);
Witepsol W25: medium-hydroxyl hard fat base;
Bleached wax: to enhance consistency;
Macrogol-8-stearate: adsorption enhancer, emulsifier;

In the following Examples, standard pharmacy equipment that is also required, e.g. a beaker, analytical balance, steel spoons, glass rod, cleaning paper, water, water bath, and drying oven, are not contained in the kit.

### Example 1: Nasal spray

### Kit description

The nasal spray kit may be designed to enable the compounding of three units of a Cannabinoid-enriched nasal spray emulsion of 15 ml per unit (≅ 15 g). With a containment of 40 % of MCT-oil-based cannabis extract, the spray offers a dosage of 10 mg of THC per ml (or 1 mg of THC per 100-micro- ml spraydose) when mixed with a 25/1 Vayamed extract. The manufacturing recipe is based on a Neostigmine Bromide nasal spray according to NRF 22.6. However, it has been optimized with a wetting agent and a pharma-grade hot-emulsifier to carry and stabilize high MCT-oil concentrations and to enhance cannabinoid take-on in the nasal mucosa. MCT (Medium Chain Triglycerides) oil contains C6 to C16 fatty acid, e.g. caprylic acid (C8) und caprinic acid (C10).

### Preparing the medicament

The kit contains all excipients, auxiliary materials, packaging materials and documentation that is needed by pharmacists to prepare the nasal spray in combination with a cannabis extract (note: the cannabis extract is not included in the kit and must come with individual prescription). Following the manufacturing instructions, pharmacists create a homogeneously stabilized cannabinoid-enriched nasal spray emulsion, which is filled into spray bottles and handed out to patients in a transport box (1 box = 3 bottles). Shelf-life can be set by pharmacists at 30 days before first use, based on a pharmacy standard for water-based preparatory solutions (given that the extract and none of the excipients show a shorter individual shelf life at the time of preparation).

### Formulation and kit items

- 3 units nasal spray bottle (20 ml, brown)
- 3 units spray-lid (100 mycro- ml spray hub, pharma-grade PU)
- 2 units of 50 ml Luer-Lock syringe
- 1 unit of 20 ml Luer-Lock syringe
- 2 units Luer-Lock inter-connector
- 1 unit Luer-Lock cap
- 1 unit Luer-Lock female connector (compatible with Vayamed extract bottle lid)
- 1 transport box in Vayamed artwork (for handout to patients)
- 1 manufacturing protocol, plausibility and identification documents
- optionally 1 brochure with dosing recommendations; product card for doctors
- Excipient (see Table 1)

**Table 1**

| Excipients Nasal Spray | Base formula | Preparatory amounts for a kit volume | Kit package sizes (not equal recipe volumes) |
|---|---|---|---|
| Preserved water | q. s. 100 % | q. s. 45 ml | 35 ml |
| Kolliphor^{®} ELP | 4% | 1.8 g | 2 g |
| Glycerol | 5 % | 2.25 g | 2.5 g |
| Xanthan gum | 0.2 % | 0.09 g | 1 g |
| Carboxymethyl cellulose (viscosity 600m Pa) | 0.2 % | 0.09 g | 1 g |
| Cannabis extract (not part of the kit) | (40%) | (18 ml) | (not included in the kit) |

### Manufacturing protocol

Time required (estimated): 20 minutes, including cooling and heating times
1. Preserved water (about 22 ml), Kolliphor ELP (1,8 g), and glycerol (2,25 g) is weighted into a beaker and mixed until a homogeneous aqueous solution is obtained.
2. Aside, carboxymethyl cellulose (0,09 g) and xanthan gum (0,09 g) are weighted.
3. The two powders from step (2) are added to the aqueous solution from step (1) and mixed well until a slightly aqueous gel is formed.
4. One of the two empty 50 ml syringes is closed with the red Luer-Lock cap. Then the gel from step (3) is poured into this syringe.
5. The smaller 20 ml syringe is prepared with the total amount of 18 ml cannabis extract by using the Luer-Lock female connector to fit into the cannabis extract bottle lid. From here, the 18 ml cannabis extract is withdrawn into the syringe.
6. The 50 ml syringe with the aqueous gel from step (4) is connected via the Luer-Lock connector to the 20 ml syringe with the cannabis extract from step (5).
7. The 20 ml syringe with the 18 ml cannabis extract is emptied into the 50 ml syringe with the aqueous gel. This leads to a total volume of 45 ml in the 50 ml syringe. The emptied 20 ml syringe is then removed. The resulting volume of liquids in the 50 ml syringe is checked again: it must be 45 ml. If not, adjust with preserved water until q. s. 45 ml is reached.
8. The other so far unused 50 ml syringe is connected to the filled 50 ml syringe from step (7), without introducing air.
9. With the Luer-Lock technique, the liquids are mixed between the two syringes by pushing the plungers back and forth. This step is repeated 30 times.
10. A milk white liquid is obtained, which is a stable nasal spray emulsion.
11. The emulsion from step (10) is filled into the 3 spray bottles, each is filled with 15 ml and closed with a spray lid.

### Example 2: Rectal suppository

### Kit description

The kit enables the compounding of ten rectal suppositories with 2 ml per unit (≅ 2 g). With a containment of 0,6 ml cannabis extract (0,3 ml/g), each suppository contains 15 mg THC when combined with a 25/1 Vayamed extract (25 mgTHC/ml). The recipe of the kit is based on a hydrophilic water-miscible fat vehicle in compliance with NRF 25.6. However, it has been optimized by adsorption enhancers and emulsifiers in order to disperse the hydrophilic vehicles into the emulsion phase more easily, to immobilize active ingredients better and to spread faster upon contact with liquid at body temperature. Typically, the suppositories dissolve within 15 minutes after application.

### Preparing the medicament

The kit contains all excipients, auxiliary materials, packaging and disposable materials that are needed by pharmacists to prepare the suppositories in combination with a cannabis extract (note: the cannabis extract is not included in the kit and comes by separate prescription). Following the manufacturing instructions, pharmacists are melting the base materials and mixing them with cannabis extract until a homogenized solution is reached, which is then filled into the suppository forms. After cooling and sealing, the forms are handed out to patients (1 box = 10 units). Shelf-life can be set by pharmacists at up-to 2 months in accordance with pharmacy standards for fat-based preparatory vehicles (given that the extract and none of the excipients have a shorter individual shelf life at time of preparation).

### Formulation and kit items

- 1 unit with 10 suppository forms (plastic molds, 2g), including a stripe of closing tap
- 2 units 35 ml Luer-Lock syringes
- 1 unit 10 ml Luer-Lock syringe
- 2 units Luer-Lock inter-connector (the 2nd one is for replacement)
- 1 unit Luer-Lock female connector (compatible to Vayamed extract bottle lid)
- 1 transport box (Vayamed artwork, for handout to patients)
- 1 manufacturing protocol, plausibility and identification documents
- optionally 1 brochure with dosing recommendations and a product card for doctors
- Excipients (see Table 2)

**Table 2**

| Excipients Rectal Suppositories | Per formula | Per kit volume | Package sizes in the kit (= plus about 10 % of recipe requirements) |
|---|---|---|---|
| Witepsol W25 | q. s. 100 % | q. s. 20 ml | 25 g |
| HPβCD | 12 % | 2,4 g | 3 g |
| Bleached wax | 3% | 0,6 g | 0,7 g |
| Cannabis extract (not part of the kit) | 0,3 ml/g | 6 ml | (not included in the kit) |

### Manufacturing protocol

Time required (estimated): 25 minutes, including cooling and heating steps.
1. Prepare a drying-oven at 40 °C. Take the Cannabis extract and withdraw 6 ml into the 10 ml Luer-Lock-syringe by using the Luer-Lock female connector to fit into the Vayamed cannabis extract bottle lid. After filing, place the syringe with the extract for about 15 minutes into the drying oven at 40 °C to warm-up the extract to avoid heat-shock during mixing in step 5.
2. While the extract from step (1) is warming-up in the drying-oven, weigh the bleached wax (0,6 g), the 2-Hydroxypropil-beta-cyclodestrin HPβCD (2,4 g) and the Witepsol (about20 g) into the same beaker by using an analytical balance.
3. Prepare a water bath at 100 °C (≅ 98 °C) and melt all components from step (2) together, without overheating (max. temp: 50 °C). After melting is complete, all components are steered well, until a homogeneous mixture is obtained.
4. Fill one of the two 35 ml Luer-lock syringes with 14 ml of the melted mixture from step (3). Connect a Luer-Lock connector to the tip of this syringe.
5. Take the syringe with the extract from step (1) out from the drying oven and connect it to the Luer-Lock adaptor at the 35 ml syringe from step (4), which contains the melted mixture. The warmed-up extract from the 10 ml-syringe is emptied into the 35 ml syringe with the melted mixture. As a result, the 35 ml syringe now carries a total volume of 20 ml of mixtures.
   Note: if the resulting volume of the liquids is below 20 ml, adjust (fill-up) with the melted mixture from step 3 until 20 ml are reached (q. s. 20 ml).
6. The emptied 10 ml syringe from step 5 is removed and the other 35 ml syringe (the empty one) is connected to the filled 35 ml syringe from step 5, without introducing air.
7. With the Luer-Lock technique, the liquids are mixed between the two syringes by pushing the plungers back and forth. This step is repeated 20 times while a milk-white liquid is reached.
   *(Note: Work fast to avoid excessive cooling inside the syringes. If the emulsion cools down before placing into the molds, one can always make it fluid again by placing the syringes on the water bath.)*
8. The 10 suppository molds are filled with the liquid from step 7, each one with 2 ml. Spilled liquid on the top of the molds is cleaned away quickly, before the liquid cools down.
9. The molds need to cool down to room temperature before they are closed with the tape stripe. With this, the suppositories are ready for handing-out to a patient.
   *(Note: After mold temperature reached 30 °C, they can be stored either at room temperature, but also in the refrigerator at 2* - *8 °C)*

### Example 3: Kit for preparing a vaginal suppository

### Kit description

The kit enables the compounding of ten vaginal suppositories with 3 ml per unit (≅ 3 g). With a containment of 0,6 ml cannabis extract (20 %, 0,2 ml/g), each suppository contains 15 mg THC when combined with a 25/1 Vayamed extract (25 mgTHC/ ml). The recipe of the kit is based on a hydrophilic water-miscible fat vehicle in compliance with NRF 25.6. However, it has been optimized by adsorption enhancers and emulsifiers in order to disperse the hydrophilic vehicles into the emulsion phase more easily, to immobilize active ingredients better and to spread faster upon contact with liquid at body temperature. Typically, the suppositories dissolve within 15 minutes after application.

### Preparing the medicament

The kit contains all excipients, auxiliary materials, packaging and disposable materials that are needed by pharmacists to prepare the suppositories in combination with a cannabis extract (note: the cannabis extract is not included in the kit and comes by separate prescription). Following the manufacturing instructions, pharmacists are melting the base materials and mixing them with cannabis extract until a homogenized solution is reached, which is then filled into the suppository forms. After cooling and sealing, the forms are handed out to patients (1 box = 10 units). Shelf-life can be set by pharmacists at up-to 2 months in accordance with pharmacy standards for fat-based preparatory vehicles (given that the extract and none of the excipients have a shorter individual shelf life at time of preparation).

### Formulation and kit items

- 1 unit with 10 suppository forms (plastic molds, 3 g), including a stripe of closing tap
- 2 units 35 ml Luer-Lock syringes
- 1 unit 10 ml Luer-Lock syringe
- 2 units Luer-Lock inter-connector (the 2nd one is for replacement)
- 1 unit Luer-Lock female connector (compatible to Vayamed extract bottle lid)
- 1 transport box (Vayamed artwork, for handout to patients)
- 1 manufacturing protocol, plausibility and identification documents
- optionally 1 brochure with dosing recommendations and a product card for doctors
- Excipients (see Table 3)

**Table 3**

| Excipients Vaginal Suppositories | Per formula | Per kit volume | Package sizes in the Kit (= plus about 10 % of recipe requirements) |
|---|---|---|---|
| Witepsol W25 | q. s. 100 % | q. s. 30 ml | 35 g |
| Bleached wax | 3% | 0,9 g | 1 g |
| Macrogol-8-stearate | 2% | 0,6 g | 0,7 g |
| Cannabis extract (not part of the kit) | 0,2 ml/g = 20 % | 6 ml | (not included in the kit) |

### Manufacturing protocol

Time required (estimated): 25 minutes, including cooling and heating steps.
1. Prepare a drying-oven at 40 °C. Take the Cannabis extract and withdraw 6 ml into the 10 ml syringe by using the Luer-Lock female connector to fit into the Vayamed cannabis extract bottle lid. After filing, place the syringe with the extract for about 15 minutes into the drying oven at 40 °C to warm-up the extract to avoid heat-shock during mixing in step 5.
2. While the extract from step (1) is warming-up in the drying-oven, weigh the bleached wax (0,9 g), the Macrogol-stearate (0,6 g) and the Witepsol (about 30 g) into the same beaker by using an analytical balance.
3. Prepare a water bath at 100 °C (≅ 98 °C) and melt all components from step (2) together, without overheating (max. temp: 50 °C). After melting is complete, all components are steered well, until a homogeneous mixture is obtained
4. Fill one of the two 35 ml syringes with 24 ml of the melted mixture from step (3). Connect a Luer-Lock connector to the tip of this syringe.
5. Take the warmed-up cannabis extract in the 10 ml syringe out from the drying oven and connect it to the Luer-Lock adaptor at the 35 ml syringe from step (4), which contains the melted mixture. The warmed-up extract is emptied into the 35 ml syringe with the melted mixture. As a result, the 35 ml syringe now carries a total volume of 30 ml of mixtures. Note: if the resulting volume of the liquids is not 30 ml, adjust (fill-up) with the melted mixture from step 3 until 30 ml are reached (q. s. 30 ml).
6. The emptied 10 ml syringe from step 5 is removed and the other 35 ml syringe (the empty one) is connected to the filled 35 ml syringe from step 5, without introducing air.
7. With the Luer-Lock technique, the liquids are mixed between the two syringes by pushing the plungers back and forth. This step is repeated 20 times while a milk-white liquid is reached.
   *(Note: Work fast to avoid excessive cooling inside the syringes. If the emulsion cools down before placing into the molds, one can always make it fluid again by placing the syringes on the water bath.)*
8. The 10 suppository molds are filled with the liquid from step 7, each one with 3 ml. Spilled liquid on the top of the molds is cleaned away quickly, before the liquid cools down.
9. The molds need to cool down to room temperature before they are closed with the tape stripe. With this, the suppositories are ready for handing-out to a patient.
   *(Note: After mold temperature reached 30 °C, they can be stored either at room temperature below 25 °C, or in the refrigerator at 2* - *8 °C).*

## Claims

1. A kit for preparing a medicament, wherein the kit does not contain an active ingredient of the medicament and contains components, equipment, and parts
for carrying out, after adding the active ingredient, a two-syringe emulsification to prepare an emulsion containing the active ingredient; and
for preparing the medicament using the emulsion.

2. The kit according to claim 1, comprising a first syringe and a second syringe, a connector for reversibly connecting the nozzles of the two syringes thereby enabling fluid communication between the two syringes, an excipient composition containing an emulsifier and being capable of forming an emulsion either with an oil-based liquid or with a water-based or water-miscible liquid, and a medicament container for accommodating an emulsion.

3. The kit according to claim 2, wherein the medicament container is selected from a nasal spray bottle, a mold for rectal suppositories, or a mold for vaginal suppositories.

4. The kit according to claim 2, wherein the excipient composition is an water-based liquid and contains 3 to 14 % by mass of a non-ionic ethoxylated vegetable oil as an emulsifier and the medicament container is a nasal spray bottle; or the excipient composition is based on a hydroxylated fat and contains 5 to 20 % by mass of 2-hydroxypropyl-β-cyclodextrin as an emulsifier and the medicament container is a mold for rectal suppositories; or the excipient composition is based on a hydroxylated fat and contains 1 to 5 % by mass of polyethylene glycol stearate as an emulsifier and the medicament container is a mold for vaginal suppositories.

5. A method of preparing a medicament comprising step (i) of providing an active ingredient and step (ii) of using the kit as described in any one of claims 1 to 4 to prepare the medicament containing the active ingredient in an emulsion.

6. The method according to claim 5, wherein the active ingredient is only available on prescription.

7. The method according to claim 6, wherein the active ingredient is or contains a cannabinoid.

8. The method according to any one of claims 5 to 7,
wherein the kit contains a first syringe, a second syringe, a connector, a medicament container, and a second liquid, step (i) of providing an active ingredient is the following step (a) of providing a first liquid containing an active ingredient, and step (ii) comprises the following steps (b) to (g); or
wherein the kit contains a first syringe, a second syringe, a connector, a medicament container, a first liquid, and a second liquid, step (i) is carried out prior to the following step (a) and comprises adding the active ingredient to the first liquid, and step (ii) comprises the following steps (b) to (g):
(a) providing the first liquid containing an active ingredient;
(b) filling the first liquid into a first syringe;
(c) providing the second liquid, wherein the second liquid is capable of forming an emulsion with the first liquid;
(d) filling the second liquid in a second syringe;
(e) using the connector to connect the nozzle of the first syringe with the nozzle of the second syringe to provide a fluid communication between the first and the second syringe;
(f) repeatedly moving the plungers of the two syringes back and forth thereby mixing the first liquid with the second liquid and forming an emulsion containing the active ingredient; and
(g) filling the emulsion containing the active ingredient into the medicament container.

9. A method of preparing a medicament containing a cannabinoid as an active ingredient, wherein the method comprises:
(a) providing a first liquid containing a cannabinoid;
(b) filling the first liquid into a first syringe;
(c) providing a second liquid capable of forming an emulsion with the first liquid;
(d) filling the second liquid in a second syringe;
(e) connecting the nozzle of the first syringe with the nozzle of the second syringe to provide a fluid communication between the first and the second syringe;
(f) repeatedly moving the plungers of the two syringes back and forth thereby mixing the first liquid with the second liquid and forming an emulsion containing the cannabinoid; and
(g) filling the emulsion containing the cannabinoid into a medicament container.

10. The method according to any one of claims 5 to 9, wherein the medicament contains 2 to 8 % by mass of non-ionic ethoxylated vegetable oil comprised as an emulsifier in the second liquid and is in the form of a nasal spray, or the medicament contains 5 to 20 % by mass of 2-hydroxypropyl-β-cyclodextrin comprised as an emulsifier in the second liquid and is in the form of a rectal suppository, or the medicament contains 1 to 5 % by mass of polyethylene glycol stearate comprised as an emulsifier in the second liquid and is in the form of a vaginal suppository.

11. A kit for preparing a medicament containing a cannabinoid, wherein
(i) the kit does not contain the cannabinoid and contains components, equipment and parts for carrying out, after adding the cannabinoid, a two-syringe emulsification to prepare the medicament containing the cannabinoid; or
(ii) the kit contains the cannabinoid and components, equipment and parts for carrying out a two-syringe emulsification to prepare the medicament containing the cannabinoid.

12. The use of a cannabinoid in a method of preparing a medicament containing the cannabinoid by carrying out a two-syringe emulsification.

13. A medicament obtainable by the method of any of claims 5 to 9.

14. A medicament containing a cannabinoid in an emulsion, wherein the medicament contains 2 to 8 % by mass of non-ionic ethoxylated vegetable oil as an emulsifier and is in the form of a nasal spray, or the medicament contains 5 to 20 % by mass of 2-hydroxypropyl-β-cyclodextrin as an emulsifier and is in the form of a rectal suppository, or the medicament contains 1 to 5 % by mass of polyethylene glycol stearate as an emulsifier and is in the form of a vaginal suppository.

15. A medicament according to claim 13 or 14 in the form of a nasal spray for use in treating pain, inflammation, Tourette syndrome, epilepsy, schizophrenia, anxiety or panic attacks, spasticity, neurodegenerative disorders, migraines, anorexia, nausea or sleep disturbances; or in the form of rectal suppository for use in treating chronic pain, anxiety or insomnia; or in the form of a vaginal suppository for use in treating endometriosis pain, menstrual related pain and cramps, dysmenorrhoea, premenstrual syndrome, sexual pain disorders, vaginismus, vulvodynia, dyspareunia, ovarian or cervical cancer pain, pelvic inflammatory disease, bacterial vaginosis, infections and inflammations.
